(19) Europäisches Patentamt
European Patent Office
Office européen des brevets

(11) Numéro de publication : **0 362 030 B1**

(12) # FASCICULE DE BREVET EUROPEEN

(45) Date de publication du fascicule du brevet : **15.07.92 Bulletin 92/29**

(51) Int. Cl.$^5$ : **A61K 7/06,** C07D 239/50

(21) Numéro de dépôt : **89402592.3**

(22) Date de dépôt : **21.09.89**

(54) **Sel du 6-pipéridino-2,4-diaminopyrimidine-3-oxyde et de l'acide acéturique, sa préparation et son application dermatocosmétologique.**

(30) Priorité : **23.09.88 FR 8812442**

(43) Date de publication de la demande : **04.04.90 Bulletin 90/14**

(45) Mention de la délivrance du brevet : **15.07.92 Bulletin 92/29**

(84) Etats contractants désignés : **AT BE CH DE ES FR GB GR IT LI LU NL SE**

(56) Documents cités :
**FR-A- 2 132 726**
**FR-A- 2 590 897**
**GB-A- 2 175 901**

(73) Titulaire : **NORCHIM**
**33, Quai d'Amont**
**F-60340 Saint Leu d'Esserent (FR)**

(72) Inventeur : **Allaigre, Jean-Pierre**
**12, rue Danton**
**F-95460 Ezanville (FR)**
Inventeur : **Desbois, Jacques**
**5, rue du Docteur E. Roux**
**F-95340 Persan (FR)**

(74) Mandataire : **Ahner, Francis et al**
**CABINET REGIMBEAU, 26, avenue Kléber**
**F-75116 Paris (FR)**

EP 0 362 030 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne l'acéturate de Minoxidil, c'est-à-dire le sel obtenu par salification du Minoxidil, ou 6-pipéridino-2,4-diamino-pyrimidine-3-oxyde, avec l'acide acéturique, ou acétyl-glycine, répondant à la formule I

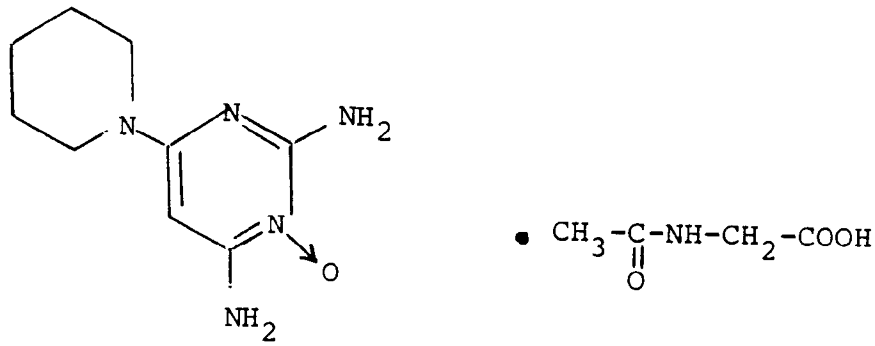

(I)

L'objet de la présente invention s'étend également au procédé de préparation de l'acéturate de Minoxidil, ainsi qu'à son utilisation en tant que principe actif dans des compositions dermatocosmétiques, en particulier dans des compositions capillaires destinées au traitement de l'alopécie.

Le Minoxidil est un composé antihypertenseur, bien connu pour son activité anti-chute des cheveux, et à ce titre est utilisé dans le traitement de l'alopécie. En raison de sa quasi-insolubilité dans l'eau,il n'est utilisé à ce jour que dans des solutions comprenant du propylène glycol et de l'alcool éthylique, 50 à 60 % en volume pour ce dernier, et donc irritantes pour un usage topique.

L'intérêt de la présente invention consiste en ce que, contrairement au Minoxidil qui est donc quasiment insoluble dans l'eau et à l'acide acéturique très peu hydrosoluble (2,7 % environ), l'acéturate de Minoxidil présente une solubilité de 34 % P/V à 20°C ce qui correspond à 21,8 % de Minoxidil actif présent dans la solution aqueuse.

Ainsi, il devient possible de préparer des solutions soit totalement aqueuses, soit contenant des quantités très réduites de propylène glycol favorable à la pénétration du cuir chevelu, mais désormais en absence d'éthanol ou tout autre solvant organique. On évite ainsi tout risque d'irritation cutanée due à la présence de ces solvents.

L'acéturate de Minoxidil peut être préparé aisément en faisant réagir le Minoxidil et l'acide acéturique, en quantités stoechiométriques en milieu aqueux ou dans un alcool aliphatique, tel que l'éthanol ou l'isopropanol. Le sel formé est isolé par précipitation ou par cristallisation. Cette synthèse peut être réalisée selon les exemples 1 et 2 ci-après.

L'acéturate de Minoxidil est un composé cristallin, chimiquement bien défini, très stable, ayant un point de fusion voisin de 155°C.

Comme le montre l'examen chromatographique en solution aqueuse, l'acéturate de Minoxidil est suffisamment dissociable pour que l'activité du Minoxidil reste inchangée.

La présente invention concerne également les formulations à usage capillaire ou pharmaceutique, contenant comme principe actif l'acéturate de Minoxidil.

Les exemples 3 et 4 proposent à titre non limitatif deux formulations possibles.

<u>EXEMPLE 1</u> :

20,9 g de Minoxidil et 11,7 g d'acide acéturique sont mis en suspension dans 500 ml d'éthanol.

Le mélange est porté à reflux pendant 1 heure.

La solution chaude est refroidie à 5°C.

Après filtration et séchage, on récupère 29,9 g d'acéturate de Minoxidil, ce qui correspond à un rendement de 92 %. Point de fusion 155°C.

Les spectre IR et RMN sont conformes à la structure.

EXEMPLE 2 :

15,2 g d'acide acéturique sont mis en suspension dans 150 ml d'eau, puis 27,2g de Minoxidil sont ajoutés sous agitation. Les cristaux se dissolvent rapidement.

La solution obtenue est concentrée au tiers de son volume, puis 650 ml d'isopropanol sont ajoutés sous vive agitation. L'acéturate de Minoxidil précipite rapidement.

Le mélange refroidi à 5°C est filtré. On récupère ainsi 37,3 g de cristaux blancs, d'acéturate de Minoxidil, ce qui correspond à un rendement de 88 %. Point de fusion : 154-157°C.

Les spectre IR et RMN sont conformes à la structure.

EXEMPLE 3 :

3,12 g d'acéturate de Minoxidil sont dissous dans 100 ml d'eau distillée.

La solution obtenue, contenant 2 g pour 100 ml de Minoxidil actif est prête pour l'utilisation à usage capillaire.

EXEMPLE 4 :

4,68 g d'acéturate de Minoxidil sont dissous dans un mélange de 120 ml d'eau distillée et 30 ml de propylène glycol.

La solution obtenue contenant l'équivalent de 2 g pour 100 ml de Minoxidil actif, est prête pour l'utilisation à usage capillaire.

Les compositions dermatocosmétiques selon l'invention peuvent affecter diverses autres formes de présentation, telles que des shampooings, gels, fixateurs ou autres lotions capillaires. Elles se sont en outre avérées très efficaces dans la pratique pour le traitement d'affections allopéciques, et ont notamment permis d'éliminer un certain nombre d'effets secondaires néfastes, tels que des irritations du cuir chevelu ou des fragilisations du cheveu par déshydratation, qui avaient été observées dans le passé lors de l'utilisation de solutions aqueuses de Minoxidil. En outre, la meilleure biodisponibilité du principe actif selon l'invention permet, avec des formulations à base aqueuse, d'abaisser la concentration de Minoxidil dissocié disponible. Certains problèmes éventuels de toxicité se trouvent ainsi parfaitement résolus, sans diminution de l'activité anti-chute.

## Revendications

1. Acéturate de Minoxidil répondant à la formule I

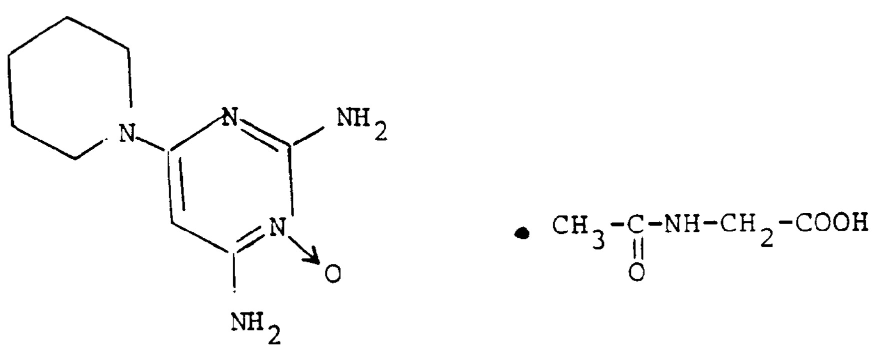

(I)

2. Procédé de préparation de l'acéturate de Minoxidil selon la revendication 1, caractérisé en ce que l'on fait réagir, en quantités stoechiométriques, le Minoxidil et l'acide acéturique en présence d'un solvant.

3. Procédé de préparation de l'acéturate de Minoxidil selon la revendication 2, caractérisé en ce que le solvant utilisé est de préférence de l'eau ou un alcool, tel que les alcools éthylique ou isopropylique.

4. Compositions dermatocosmétiques, caractérisées en ce qu'elles contiennent en tant que principe actif l'acéturate de Minoxidil selon la revendication 1, en mélange avec un support à base aqueuse.

## Patentansprüche

1. Minoxidil-Aceturat nach der Formel I

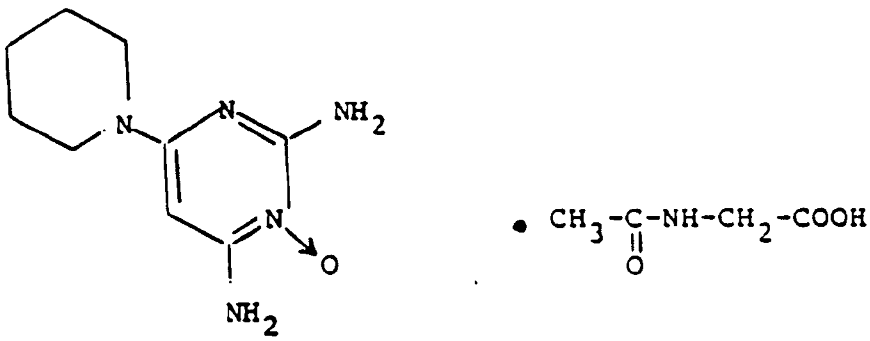

(I)

2. Verfahren zur Herstellung des Minoxidil-Aceturats nach Anspruch 1, dadurch gekennzeichnet, daß man das Minoxidil und die Acetursäure, in Stoechiometrischem Mengen, in der Gegenwart eines Lösungsmittels miteinander reagieren läßt.

3. Verfahren zur Herstellung des Minoxidil-Aceturats nach Anspruch 2, dadurch gekennzeichnet, daß das verwendete Lösungsmittel vorzugsweise Wasser oder Alkohol ist, insbesondere Ethyl- oder Isopropylalkohol.

4. Dermatokosmetische Verbindungen, dadurch gekennzeichnet, daß sie als Wirkstoff das Minoxidil-Aceturat nach Anspruch 1 enthalten, in Verbindung mit einer wässrigen Lauge als Trägerstoff.

## Claims

1. Minoxidil aceturate corresponding to the formula I

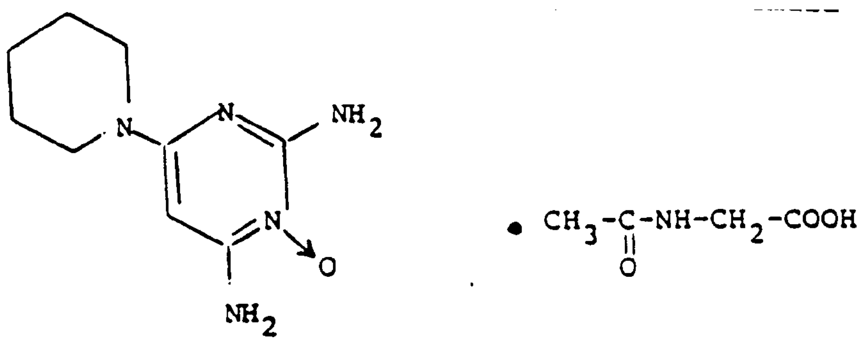

(I)

2. A process for preparing minoxidil aceturate as claimed in claim 1, wherein minoxidil and aceturic acid are reacted in stoichiometric amounts in the presence of a solvent.

3. The process for preparing minoxidil aceturate as claimed in claim 2, wherein the solvent used is preferably water or an alcohol such as ethyl or isopropyl alcohol.

4. Dermato-cosmetic compositions, which contain minoxidil aceturate as claimed in claim 1 as active principle, mixed with an aqueous-based vehicle.